# NEW EUROPEAN PATENT SPECIFICATION

(11) **EP 2 601 203 B2**
(45) Date of publication and mention of the opposition decision: **25.03.2020**
(45) Mention of the grant of the patent: 08.02.2017
(21) Application number: 11752317.5
(22) Date of filing: 02.08.2011
(51) Int. Cl.: C07H 13/06, C07H 15/10, C12N 1/20, C12P 23/00, C12Q 1/02, C12R 1/07, C09B 61/00, A61K 35/74, A61K 39/07, A61K 8/30, A61K 8/96, A61Q 19/08, A23L 3/3472, A23L 3/3562, A23L 2/58, A61K 35/742, C12P 19/44, A23K 10/16, A23K 20/179

(54) **FATTY ACID ESTERS OF CAROTENOID GLUCOSIDES AS COLOURING AGENTS FOR FOODSTUFFS**
FETTSÄUREESTER VON KAROTINOID GLUKOSIDEN ALS FÄRBEMITTEL FÜR LEBENSMITTEL
ESTERS D'ACIDE GRAS DE GLUCOSIDES CAROTENOIDES EN TANT QU' AGENTS COLORANTS POUR DES PRODUITS ALIMENTAIRES

(30) Priority: 03.08.2010 GB 201013041
(43) Date of publication of application: 12.06.2013
(73) Proprietor: Royal Holloway And Bedford New College, Egham, Surrey TW20 0EX (GB)
(72) Inventor: FRASER, Paul David, Egham Surrey TW20 0EX (GB); FONS, Laura Perez, Egham Surrey TW20 0EX (GB)
(74) Representative: Schlich, George
(86) International application number: PCT/GB2011/001159
(87) International publication number: WO 2012/017199

(56) References cited:
- WO-A2-2009/126890
- KAZUTOSHI SHINDO ET AL: "Methyl Glucosyl-3,4-dehydro-apo-8'-lycopenoate, a Novel Antioxidative Glyco-C30-carotenoic Acid Produced by a Marine Bacterium Planococcus maritimus", THE JOURNAL OF ANTIBIOTICS, vol. 61, no. 12, 1 December 2008 (2008-12-01), pages 729-735, XP55008116, ISSN: 0021-8820, DOI: 10.1038/ja.2008.86
- ROLF JANSEN ET AL: "Four new carotenoids fromPolyangium fumosum (myxobacteria): 3,3',4,4'-tetradehydro-1,1',2,2'-tetrahydr o-1,1'-dihydroxy-[Psi],[Psi]-carotene (di-O-demethylspirilloxanthin), its [beta]-glucoside and glucoside fatty acid esters", LIEBIGS ANNALEN, vol. 1995, no. 5, 1 May 1995 (1995-05-01), pages 873-876, XP55008109, ISSN: 0947-3440, DOI: 10.1002/jlac.1995199505126
- P.D. FRASER et al.: "Application of high-performance liquid chromatography with photodiode array detection to the metabolic profiling of plant isoprenoids", Plant J, vol. 24, no. 4, 2000, pages 551-558,
- P.D. FRASER et al.: "Metabolite profiling of plant carotenoids using the matrix-assisted laser desorption ionization time-of-flight mass spectrometry", Plant J, vol. 49, 2007, pages 552-564,

## Description

The present invention relates to uses of carotenoids and novel carotenoid molecules. In particular, the invention relates to carotenoid molecules and their uses, such as uses as colouring agents in edible matter and as components in pro-biotic compositions suitable for use in edible matter, such as foodstuffs, pro-biotic yoghurts, and pro-biotic drinks, and further uses in cosmetics.

To date over 700 structures have been reported for carotenoids isolated from plants, fungi and bacteria [1]. A defining feature of carotenoids is their chromophore, which consists of a series of conjugated double bonds. It is the conjugated bonds that confers colour to the molecule. The ability of carotenoids to confer colour to foodstuffs has fuelled commercial interest in these molecules, for example, for their use as natural colouring agents. In addition to their utility as colouring agents many carotenoids are considered to have health promoting properties. For example, the dietary intake of lycopene-containing products is associated with the prevention of, and more recently the treatment of, certain cancers such as prostate cancer [2-4]; while β-carotene, also known as provitamin A, is a dietary component that is essential for human health [5,6]. Carotenoids possessing a C40 backbone have been the subject of commercial attention because of their availability in nature and ability to be exploited. One example of a commercially available C40 bicyclic carotenoid is canthaxanthin, with an annual sales market of US$280 million; it is used as a feed supplement in the aquaculture and poultry industries [7].

Carotenoids for use in industry are typically obtained by chemical synthesis. However, high production costs, the dependence on fossil fuels for energy and for the supply of suitable reactant organic molecular species, and publicly perceived detrimental impacts on the environment have resulted in intensified efforts to find alternative production methods, particularly from biological sources.

Despite their intense colour, the disadvantages of using conventional carotenoids as natural colouring agents include their poor solubility in aqueous solutions, and instability therein. Such properties tend to be problematic when using conventional C40 backbone carotenoids because of their hydrophobic chemical structure.

Certain bacteria are able to produce a diverse range of carotenoids with both C40 and C30 backbones. The carotenoids with a C30 backbone are often referred to as diapocarotenoids and are typically found in a limited number of gram positive (gram +ve) bacteria such as *Methyobacterium rhodinum* (formerly *Pseudomonas rhodos*), [8-10], *Streptococcus faecium* [11], *Heliobacterium* [12,13], and *Staphylococcus* [14-16].

WO 2007/066108 describes the provision of non-pathogenic spore-forming *Bacilli* and their use in foodstuffs. However, WO 2007/066108 is silent as to the identity and suitability of C30 diapocarotenoids of the present invention that may be of use in a commercial context, such as in human foodstuffs or in non-human animal feedstuffs. WO 2007/066108 also refers to *Bacilli* strains that have been deposited with the NCIMB, such as HU 36 (NCIMB No. 41361), referred to in the experimental section of the present application. WO 2009/126890 discloses a recombinant fungus and a method of preparing a food or feed additive containing a carotenoid having a 29 carbon carotenoid backbone. C30 diapocarotenoids of the invention possessing defined fatty acid constituents at R₂ are not referred to in WO 2009/126890. Shindo K. et al The Journal of Antibiotics, vol. 61, No.12, 1 December, 2008, pages 729-735 describes a C30 structure having a CH₂OH group attached to the glucosyl moiety which is not present in the structures encompassed by the instant invention, Furthermore, there is no reference to the compounds described by Shindo et al being contemplated for use as colouring agents for foodstuffs.

The inventors have now isolated diapocarotenoid molecules (carotenoids possessing C30 backbones) from spore forming *Bacillus spp.* that are capable of being used in the food industry. The diapocarotenoids of the invention possess better stability in aqueous environments and are soluble in such environments whereas C40 carotenoids, such as lycopene, are substantially insoluble in aqueous environments. The diapocarotenoids of the invention have been found to possess an antioxidant capability that is similar to or better than that of lycopene despite having a similar number of double bonds, and this was unexpected. Furthermore, the diapocarotenoid-containing spores of such *Bacillus spp.* also have use in the food industry as well as the *Bacillus spp.* themselves, which may also be used in probiotic applications [17].

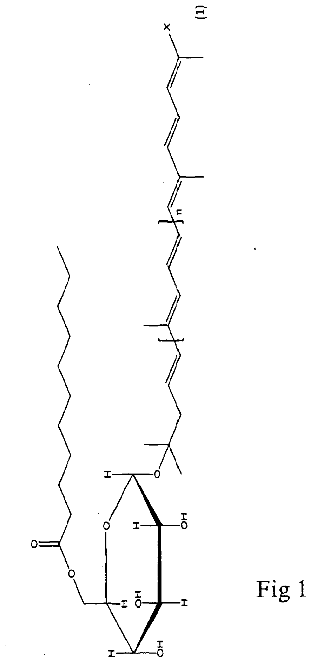

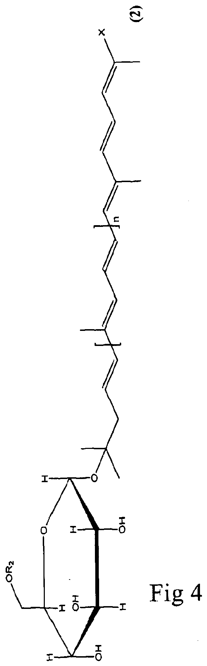

According to the present invention there is provided a carotenoid compound of Formula (2) as shown in Figure 4 wherein X is methyl or COOR wherein R is independently selected from methyl, ethyl, methylethyl, propyl, isopropyl, butyl, isobutyl, sec-butyl, and tert-butyl; and n is selected from zero, 1, 2, and 3; and R₂ is independently selected from octadecanoic acid, nonadecanoic acid, decanoic acid, undecanoic acid, dodecanoic acid, tridecanoic acid, tetradecanoic acid and pentadecanoic acid. Preferred carotenoid compounds of the invention are those of Formula (1) as shown in Figure 1 wherein X is methyl or COOR wherein R is independently selected from methyl, ethyl, methylethyl, propyl, isopropyl, butyl, isobutyl, sec-butyl, and *tert*-butyl; and n is selected from zero, 1, 2, and 3. Preferably, a compound of Formula (1) is one where X is methyl and n is 3, or X is COOR wherein R is methyl, and n is 3. Compounds of Formula (1) are typically orange or yellow in colour and are stable up to a temperature of about 70°C. Such compounds of Formula(1) have use as food additives in foodstuffs that are prepared in conditions wherein the temperature is not permitted to go above about 70°C and more preferably, where the temperature is not permitted to go above about 60°C.

The person skilled in the art will appreciate that the R group of Formula (1) may be modified by way of chemical modification of the carboxyl group to form esters. Such esters may be formed by reacting the carboxyl group with the corresponding alcohol (methanol, ethanol, butanol, propanol, isopropanol, etc.) in the presence of a proton acid catalyst such as hydrogen chloride or sulphuric acid (Fischer esterification). The reaction may be carried out according to standard chemistry protocols, for example, such as that described by Laurence M. Harwood and Christopher J. Moody in "experimental Organic Chemistry, Principles and Practice" (1989), Blackwell Science, Ltd.. Furthermore, the person skilled in the art will appreciate that other methods such as by way of enzymic modification of the structure where R is as defined for -COOR in Formula (1) may also be performed using non-specific acyltransferases (EC 2.3) in the presence or absence of acetyl CoA. Such carotenoid-modifying enzymes may be isolated from and purified from plant tissues having a high carotenoid content, using protocols and procedures known in the art. Typically, plant tissues rich in carotenoids may be selected from flowers and fruits. Glycosyltransferases (EC. 2.4) can be used to modify the sugar content of the molecule using sugar nucleotide donors e.g. UDP-glucose, UDP-galactose, UDP-GlcNAc, GDP-mannose, UDP-GalNAc, UDP-xylose, UDP-glucuronic acid and GDP-fucose.

In a further aspect of the invention there is provided use of at least one isolated compound of Formula (2) or Formula (1) as a colouring agent for a foodstuff. Foodstuffs that include isolated compounds of Formula (1) are typically coloured more brightly than comparable foodstuffs and as a result of containing such compounds may have a longer shelf life than conventional foodstuffs that do not contain isolated compounds of the invention. The isolated compounds of Formula (1) of the invention also possess antioxidant activity and are thought to function in part to slow down oxidation of foodstuffs that contain them. Thus, as a further aspect of the invention, there is provided a foodstuff that comprises at least one isolated compound of Formula (1). The foodstuff may include two, three, four or more isolated compounds of Formula (1) depending on design. For the purposes of the present invention, a foodstuff may be a beverage, a non-liquid foodstuff or may make up a supplement that is incorporated into a foodstuff. A foodstuff is typically made up of one or more of protein, carbohydrate and fat. The foodstuff may contain one or more micronutrients such as vitamins or minerals and the like. Examples of foodstuffs that may incorporate isolated compounds of Formula (1) include snack bars, cereals, dips and spreads, confectionery, probiotic formulations including yoghurts, and frozen confections, cheeses, plant-oil or plant-fat based fluids, animal fat-based fluids and other dairy products. Preferred foodstuffs incorporating isolated compounds of Formula (1) include yoghurts, cheeses, soft beverages such as squashes (e.g. orange and lemon), dry drink mixes, nutritional beverages and teas. The beverage may be alcoholic and the isolated compounds of Formula (1) may be added to introduce colour. Further kinds of foodstuff, including aquatic and marine animals that are destined to be a foodstuff for human or non-human consumption and which may be fed a diet containing isolated compounds of Formula (1), may be found in WO2007/066108.

In other applications, for example, the addition of compounds of Formula (1) to foodstuffs that are processed at temperatures higher than about 70°C, such compounds may be added in the form of isolated spores of *Bacillus* spp. comprising compounds of Formula (1). The advantage of adding spores of *Bacillus* spp. comprising compounds of Formula (1) is that when incorporated in the spore, the compounds of Formula (1) display a significant colour stability to heat relative to conventional colouring agents such as β-carotene that are employed in the art, and may be subjected to higher temperatures, such as temperatures of up to about 200°C without altering the apparent colouring of the product (as detectable by the naked eye) in which they occur. Products such as foodstuffs comprising spores do not appear to suffer from substantial detriment in colour, and hence the perceived colour thereof. Such spores may find uses in the colouring of food mixes for raw, processed foodstuffs that are then cooked such as buns, muffins, biscuits, cakes, pastries, processed vegetables, sweets (candies), processed sauces and the like.

In addition to being used in or as foodstuffs primarily destined for human consumption, the person skilled in the art will appreciate that the diapocarotenoids of the invention may be used as components or ingredients of non-human, animal feedstuffs. Thus, as a further aspect of the invention, there is provided an animal feedstuff comprising compounds of the invention. Such feedstuffs typically comprise pelleted food, such as that provided to farmed animals such as pigs, cattle, horses and poultry such as hens, turkeys and guinea fowl and the like. Such animal feedstuffs may comprise diapocarotenoid compounds of the invention that have been added *per se.*

Diapocarotenoid compounds of the invention may also find use in formulations for the cosmetic industry, such as in topically applied formulations including make-ups, anti-aging formulations, e.g., creams for fine lines and/or wrinkles, skin penetration enhancers, sprays, and the like. The diapocarotenoid compounds of the invention comprised in cosmetic compositions can be formulated in a variety of product forms. Preferably, the compositions are prepared in targeted delivery systems, e.g. creams, lotions, gels, and the like, particularly for topical administration.

Compositions containing diapocarotenoid compounds *per se* can be formulated into carrier vehicles such as liposomes which may comprise other additives commonly used in the cosmetics industry. Preferably, diapocarotenoid compounds of the invention are in a cosmetically or dermatologically acceptable formulation that is suitable for contacting with the human skin. Formulations comprising diapocarotenoids of the invention may be in any cosmetically and/or dermatologically suitable form such as a lotion or cream, an anhydrous or aqueous base, or in a sprayable liquid form. Other suitable cosmetic product forms include, for example, an emulsion, a lip balm, a lip gloss, a lotion, a mask, an ointment, a mousse, a patch, a pomade, a solution, a spray, a wax-based stick, or a towelette. Naturally, the person skilled in the art will appreciate that cosmetic compositions comprising diapocarotenoid compounds of the invention may include one or more compatible cosmetically acceptable adjuvants commonly used in the art, such as fragrances, emollients, humectants, preservatives, vitamins, chelators, thickeners, anaesthetics, anti-allergenics, anti-fungals, antimicrobials, other anti-inflammatory agents, further antioxidants, antiseptics, de-pigmenting agents, film formers, insect repellents, pharmaceutical agents, photo-stabilizing agents, sunscreens, stabilizers, surfactants, thickeners, viscosity modifiers, and other ingredients such as botanicals selected from aloe, chamomile, and the like.

Cosmetic compositions of the invention comprise diapocarotenoid compounds of the invention *per se,* such as compounds of Formula (1) and/or of Formula (2). Such cosmetic compositions include diapocarotenoid compounds of the invention.

In a further aspect of the invention, there is provided a foodstuff comprising compounds of the invention of *Bacillus spp.*

In a further aspect of the invention, there is provided use of a compound of Formula (2) as depicted in Figure 4 in a food stuff or in an animal feedstuff wherein R₂ is H ; and X, COOR and n are all as defined as for compounds of Formula (1). Such compounds as defined under Formula (4) are believed to exhibit good gastric stability and hence find use as antioxidants in the diet or as a source of supplemental antioxidants in the diet. As a result, such compounds make useful additions to the diet because they are thought to be useful in helping to prevent the onset of disease, or indeed of ameliorating symptoms of disease. *Bacilli* extracts comprised of compounds of the invention may be used in the manufacture of medicaments for treating or preventing the onset of diseases such as cancers, heart disease, atherosclerosis, cataracts, and macular degeneration of the eye, stroke, dementia, Alzheimer's disease, osteoporosis, and chronic fatigue syndrome. Examples of cancers against which compounds of the invention may have application include lung cancer, breast cancer, prostate cancer, and colorectal cancer, in particular lung and prostate cancer. Compounds of the invention may be used in the manufacture of medicaments for treating wrinkling of the skin, and other characteristics of ageing; to treat diabetes in pre- and post-presentation of physiological symptoms of diabetes.

As a further aspect of the invention, there is provided at least one compound of the invention when used as a colouring agent in a foodstuff. Furthermore, there is provided at least one compound of the invention when used as an antioxidant in a foodstuff.

The provision of compounds of the invention enables the manufacture of a medicament, food, food supplement, probiotic, nutraceutical, or dietary supplement for improving any of the conditions mentioned herein. The compounds of the invention are typically gastric resistant and as such, lower doses of compounds of the invention may be employed than conventional doses of other medicaments for a similar benefit.

A nutraceutical is a food ingredient, a food supplement or a food product which is considered to provide a measurable medical or health benefit. Generally-speaking, a nutraceutical is specifically adapted to confer a particular health benefit on a subject consumer. A functional food is typically marketed as a providing a health benefit beyond that of supplying pure nutrition to the consumer.

Compositions comprising compounds of the invention may be in diverse forms, for example, in the form of a tablet, a capsule or a powder. When the composition is in the form of a powder, it may be provided in an air-tight container such as a sachet or a bottle.

Examples of excipients which may be present in the various compositions that comprise compounds of the invention include diluents (e.g. starch or cellulose derivatives a sugar derivative such as sucrose, lactose or dextrose), a stabilizer (e.g. a hygroscopic component such as silica or maltodextrin), a binder, buffer (e.g. a phosphate buffer), a lubricant (e.g. magnesium stearate), coating agent, preservative, emulsifier dye, flavouring, and/or suspension agent. Suitable excipients are well known to those skilled in the art.

Products of the invention comprising compounds of the invention may comprise a carrier or excipient which may be a solvent, dispersion medium, coating, isotonic or absorption delaying agent, sweetener or the like. Suitable carriers may be prepared from a wide range of materials including but not limited to diluents, binders, and adhesives, lubricants, disintegrants, further colouring agents, bulking agents, or the like that may be required in order to prepare a particular dosage form.

Solid oral dosage forms may contain together with one or more active compounds of the invention, diluents such as silica, talc, stearic acid, magnesium, or calcium stearate and/or polyethylene glycols; binding agents such as starches, Arabic gums, gelatin, methylcellulose, carboxymethylcellulose, or polyvinyl pyrrolidone; disintegrants such as starch, alginic acid, alginates or sodium starch glycolate; effervescing mixtures; dyestuffs, sweeteners; wetting agents such as lecithin, polysorbates, lauryl sulphates, and the like. Such preparations may be manufactured in known ways, for example by means of mixing, granulating, tabletting, sugar coating, or film coating processes.

Liquid dispersions for oral administration may be syrups, emulsions, and suspensions. Syrups may contain as carrier, for example, saccharose, or saccharose with glycerol and/or mannitol and/or sorbitol. The suspensions and the emulsions may contain as carrier, for example, a natural gum, such as guar gum, agar, sodium alginate, pectin, methylcellulose, carboxymethylcellulose, or polyvinyl alcohol. As the diapocarotenoids of the invention are water soluble, appropriate formulation will be employed taking into account their water solubility.

Suitable types of formulation may be found in Remington's Pharmaceutical Sciences, Mack Publishing Company, Eastern Pennsylvania, 17th Ed. 1985.

There now follow non-limiting examples and figures illustrating the invention. It is to be understood that the examples are not to be construed as limiting the scope of the invention in any way.

### Legends to Figures

**Figure 1****:** Compound of Formula (1)
**Figure 2****:** Methyl 1-(6-C11:0)-glycosyl-3,4-dehydro-apo-8'lycopenoate (orange)

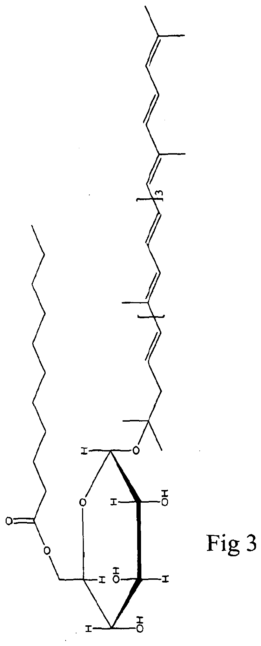

**Figure 3****:** 1-(6-C11:0)-glycosyl-3,4-dehydro-apo-8'-lycopene (yellow)
**Figure 4****:** Compound of Formula (2)
**Figure 5****:** HPLC traces at 286 nm (A) and 450 nm of unsaponified extract of HU36 freeze-dried cells (B) and after saponification with 10% NaOH (C). Chromatographic peaks were grouped according to their UV/Vis spectra (I-IV) showed in panel D.
**Figure 6****:** Characteristic MS spectra of major carotenoids found in saponified and not-saponified extracts of HU36 (A-E) and their corresponding structures and fragmentation patterns
**Figure 7****:** HPLC chromatograms recorded at 286 nm (A) and 350 nm (B) of HU36 cells grown on TY agar plates treated with 100 µM of the desaturase inhibitor diphenylamine (DPA). Peaks 1 and 2 represent the first carotenoid precursor of the pathway apo-8'-phytoene and its geometric isomer. The following intermediates of the biosynthesis apo-8'-phytofluene and apo-8'-ζ-carotene are indicated by peaks 3-5 and 6, respectively. Quantification of coloured pigments glycosyl-apolycopene and methyl glycosyl-apolycopenoate and the colourless precursor apophytoene is shown in figure C.
**Figure 8****:** HPLC chromatograms recorded at 450 nm showing the carotenoid profile of HU36 grown on liquid DSM medium and harvested at different sporulation stages: 24 hr (A) and 72 hr (B). Peak 1 represent the orange pigment associated to sporulation, methyl glycosyl-apo-8'-lycopenoate, and peak 2 correspond to the yellow pigment glycosyl-apo-8'-lycopene. Quantification by HPLC-PDA of major peaks are shown in figure C.
**Figure 9****:** HPLC chromatograms recorded at 450 nm showing the carotenoid profile of HU36 grown on liquid TY medium and harvested at different time-points: 24 hr (A) and 72 hr (B). Peak 1 and 2 represent the orange and yellow pigments respectively, methyl glycosyl-apo-8'-lycopenoate and glycosyl-apo-8'-lycopene. In TY medium an additional peak eluting at 8 min was detected showing a characteristic absorption spectrum at 436, 466 nm. Quantification by HPLC-PDA of major peaks are shown in figure C.

### Examples Section

### 1. Materials and methods

### 1.1. Bacterial strains and cultivation

The yellow and orange pigmented *Bacillus* spp. used in this study have been described previously [18]. To generate biomass for the structural elucidation of pigments *Bacillus indicus* HU36 was used routinely. General cultivation was carried out in LB media on agar 48 hr at 30°C or in shake culture for 48 hr, 30°C at 200 rpm. Developmental staging of growth was performed with either tryptone-yeast extract (TY) for vegetative cell growth or Difco Sporulation Media (DSM) for the generation of spores [19].

### 1.2. Determination of sporulation efficency and time-course experiments.

From a master glycerol stock DSM agar plates were inoculated with a HU36 streak and incubated for 2 days at 30°C. Following colony growth a single colony was used to inoculate DSM liquid media (10 ml). The culture was incubated at 30°C and agitated at 200 rpm, until the mid-exponential phase of growth was reached (OD₆₀₀, 0.6 AU). An aliquot (1 ml) of this culture was then used to seed DSM liquid media (50 ml) present in 250 ml baffled flasks. These cultures were then incubated at 30°C, 200 rpm for three days.

To determine the percentage of sporulation over growth and development, the viable counting of vegetative cells and heat-resistant spores was carried out. Data points were collected every 24 hrs from inoculation. At each time point a culture aliquot (0.5 ml) was taken and 0.1 ml used to make a serial dilution in phosphate buffer (0.85% (w/v) pH 7). Each dilution was plated onto DSM agar and after 24 hrs at 37°C colonies were counted. The remaining 0.4 ml of culture was heated at 65°C for 1 hr to eliminate vegetative cells and retain heat-resistant spores. Serial dilutions of the heat treated solution were then carried out. The data was expressed as colony forming units (c.f.u) per ml and the sporulation efficiency provided as a percentage from the number of heat resistant spores/the number of vegetative cells. To ascertain the sporulation state cultures were examined microscopically. Material for carotenoid analysis was obtained every 24 hrs from inoculation; the cellular material was harvested by centrifugation at 10,000 g performed at 4°C for 10 min. The pelleted cells were freeze-dried and stored at -80°C.

### 1.3. Carotenoid extraction, separation, identification and quantification.

Material for carotenoid analysis was prepared by growing *Bacillus spp.* in baffled flasks (2 L) containing TY liquid media (500 ml), incubated at 30°C and agitated at 200 rpm. Cells were harvest from the media after 2 to 3 days growing by centrifugation. The cellular biomass was frozen and lyophilized to completed dryness (about 3 days). This material was either used immediately or stored at-80°C. A homogeneous powder was prepared using a mortar and pestle or tissue lyser (Qiagen, Crawley, UK). An aliquot (50 mg) of the homogenised freeze-dried material was taken for extraction. The extraction of carotenoids from this material was performed with and without saponification. Firstly, direct extraction without saponification was carried out with methanol (2 ml), added to the powdered cellular material. The suspension created was then sonicated at room temperature for 10 to 15 min. Centrifugation (12,000 rpm) for 3 mins was carried out to remove the cell debris and create the carotenoid containing supernatant. The cellular debris was re-extracted until no extractable colour was recovered from the cellular material. The methanolic extracts were pooled and then dried under vacuum (centrifugal evaporator EZ-2 Plus, Genevac, Ipswich, Suffolk, UK) and the dried carotenoid extract was stored at -20°C under nitrogen for further analysis.

Saponification prior to extraction was performed by treating the freeze-dried cells (50 mg) with a solution of NaOH (10% w/v; 1 ml) and sonicating the suspension for 15 - 20 mins at room temperature. The NaOH was removed by centrifugation and from the digested cellular material methanol (250 µl) and chloroform (500 µl) was added, mixed and then a partition created with Tris buffered saline (50 mM Tris-HCl, 1 M, pH 7.0). After centrifugation the carotenoid-yielding hypo phase was removed and the aqueous hyper phase re-extracted with chloroform (500 µl) twice (at this point no colour resides in the cellular debris). The pooled organic extracts were reduced to dryness under a stream of nitrogen gas; typically these dried extracts were stored at -20°C, for further analysis.

Due to their lack of solubility in chloroform or ethyl acetate, dried extracts were routinely dissolved in chloroform:methanol (1:1 by vol.). TLC separations of these *Bacillus* derived carotenoids were carried out using two systems. Firstly, system I comprised of an activated silica gel stationary phase and the mobile phase acetone (35%) in petroleum ether (b.p. 80-100°C [15] and system II an identical stationary phase with toluene/ethyl acetate/methanol (50:25:25 by vol.) [20].

For chemical modification, the non-hydrolysed carotenoid extracts were dissolved in methanol containing 10% KOH and heated for 20 min at 60°C. They were further fractioned by direct partitioning into ether (alkaline fraction) followed by pH adjustment to 3 with HCl and partitioning into ether again (acid fraction).

High Performance Liquid Chromatography (HPLC)-Photodiode array (PDA) analysis of carotenoids was performed with a Waters Alliance (Milford, MA) 2600S system. Detection was carried out with an online PDA. Separations were performed on a reverse phase (RP) C₃₀ 5µm column (250 x 4.6 mm i.d.) with a C₃₀ guard column (20 x 4.6 mm), (YMC Inc., Wilmington, NC), which were maintained at 25°C. Prior to injection onto the column extracts were filtered through a PTFE membrane (0.2 µm: Chromacol Ltd., Herts, UK.) and then centrifuged at 12,000 rpm for 3 min. The mobile phase used for routine analysis comprised of (A) methanol, (B) methanol [21]: water (80:20 by vol.) containing 0.2% (w/v) ammonium acetate and (C) *tert*-butyl methyl ether. Elution from the column was carried out from 95% (A) and 5% (B) for 12 min, then a step to 80% (A), 5% (B) and 15% (C) followed by a linear gradient to 30% A, 5% B and 65% C at 30min. The column was then returned to the initial conditions and equilibrated over 30 min. A flow rate of 1 ml/min was employed and elution monitored continuously with the on-line PDA (200 to 600 nm). Identification was performed by the comparison of spectral and chromatographic characteristics to authentic and similar carotenoids as well as reference parameters in the literature **[17, 22].** Quantification was carried out using dose-response curves prepared from authentic standards previously purified by HPLC. For purification saponified extracts were separated by HPLC using identical conditions as described above and pure compounds isolated using a fraction collector. Absorption coefficients (ε) calculated for both yellow and orange pigments were 149621 and 122300 (M⁻¹ cm⁻¹), respectively **[22, 23].** Menaquinone was also indentified by spectral comparison with authentic standards. All solvents were purchased from VWR (Poole, UK).

To complement the UV/Vis and chromatographic properties used to identify the carotenoids in question, Mass Spectrometry (MS) was also employed. Separations were performed by HPLC prior to on-line MS in a similar manner to that detailed above, with the exception that a RP C₃₀ 3 µm column (150 x 2.1 mm i.d.) coupled to a 20 x 4.6 mm C₃₀ guard column was used. The mobile phase was altered to facilitate ionisation and comprised of (A) methanol containing 0.1% formic acid (by vol.) and (B) *tert*-butyl methyl ether containing 0.1% formic acid (by vol.). These solvents were used in a gradient mode starting from 100% (A) for 5 min, then stepped to 95% (A) and 5% (B) for 4 mins, followed by a linear gradient over 30 min to 75%(B) reducing buffer (A) accordingly to 25% (A). After this gradient, (A) was stepped down to 10% for 10 min and initial conditions (100% A) were restored for 10 min after the gradient to re-equilibrate the system. The flow rate used was 0.2 ml/min. The ionisation mode employed was Atmospheric Pressure Chemical Ionisation (APCI) operating in positive mode (Thermo Scientific, San Jose, California, USA). Capillary and APCI vaporiser temperatures were set at 225°C and 450°C respectively and the gas flow (nitrogen) at 80 units. APCI source settings were as follows; source voltage at 4.5 kV, source current 5 µA and a capillary voltage of 3 V. A full MS scan was performed from 300 to 1500 m/z and MS/MS spectra were recorded at a normalised collision energy of 35% and isolation width of 1 m/z.

### 2. Results and Discussion

### 2.1. Isolation of carotenoid pigments from Bacillus material

*Bacillus spp.* producing yellow and orange carotenoids have been genotyped **[18].** Of particular interest is the *Bacillus indicus* HU36 strain and related species due to their amenable probiotic properties **[17].** The yellow/orange carotenoids formed by these species are the focus of the present study.

When bacillus species were grown on either solid or in liquid TY or DSM-based media carotenoids were produced. It was evident that the type and quantity of carotenoids produced varied with the culture conditions used. However, growth in TY broth for 48 hr resulted in a comparatively higher biomass (5-fold and 3-fold increase at days 2 and 3, respectively) with a carotenoid composition that was representative of all the culture conditions assessed. Thus this procedure was standardised for the generation of material to be used in the structural elucidation of the carotenoids that were present. The carotenoids produced were retained in an exclusive manner within the cellular pellet; no carotenoids were present in the spent media. Extractions from fresh, frozen and freeze-dried material were evaluated in conjunction with different solvent treatments (e.g. methanol, acetone, chloroform, diethyl ether, DMSO and ethyl acetate) and homogenisation procedures included French press, manual grinding with liquid nitrogen, tissue lysers and sonication. Methanol extraction of ground freeze-dried material was comparatively-speaking the most effective approach but large volumes and multiple re-extractions with methanol were required. Alternatively, rapid chemical treatment with 10% NaOH at room temperature followed by sonication released the carotenoids into CHCl₃:CH₃OH (2:1). The carotenoids could then be enriched into CHCl₃ phase with the addition of buffer. The profile and UV/Vis spectra of the carotenoids extracted with methanol solely and CHCl₃ were identical. The chemical treatment procedure used represented an efficient extraction procedure that did not modify the structure of the native carotenoids present.

### 2.2. The separation and identification of Bacillus carotenoids

Glycosidated C30 carotenoids have been reported previously in some gram +ve bacteria such as *Staphylococcus aureus, Planococcus maritimus* and *Heliobacillus.* Therefore the separation of *Bacillus* pigments was initially performed on thin layer chromatography (TLC), using systems devised for these triterpenoids and their glycosides. TLC system I revealed both yellow and orange *Bacillus* pigments with R_{f} values of 0.95 and 0.91 respectively, indicating that the orange pigment was more polar. The yellow and orange pigments were designated C455 (compound 19) and C467 (compound 15) respectively according to their adsorption maxima in the visible range. Following saponification with KOH the C455 carotenoid accumulated in an alkaline ether fraction, whereas the C467 product was found in the acidified methanolic fraction. TLC analysis on a system suitable for glycosyl diapocarotenoic acid derivatives indicated that the C455 hydrolysis product had an R_{f} of 0.56 being less polar than the C467 product which had an R_{f} of 0.50. The non-hydrolyzed carotenoids remained on the origin. Table 1 A and B summarises the chromatographic behaviour of the *Bacillus* pigments on both TLC systems used and prior to and after saponification.

**Table 1: Chromatographic and spectral properties of detected in not-saponified extract of HU36 analysed by TLC, HPLC-PDA and LC/MS. Y: yellow (glycosyl-apolycopene): LY: light yellow; OR: orange carotenoid (methyl glycosyl-apolycoponoate); NA: not applicable Nd: not detected.**

| | **TLC analysis** | | **HPLC** | **Spectral Properties** | | | |
|---|---|---|---|---|---|---|---|
| **Carotenoid** | **Rf** | **Colour** | **Rt** | **λmax (nm)** | **%III/II** | **%AB/AII** | **[M+H]+** |
| **Trace at 286 nm, saponified and unsaponified extracts (****Fig. 5A****):** | | | | | | | |
| Apo-8'-phytoene (1) | - | White | 9.49 | 274, 286, 298 | <1 | NA | 409.20 |
| Apo-8'-phytoene isomer (2) | - | White | 10.35 | 274, 286, 298 | <1 | NA | 409.20 |
| Menaquinone-7 (3) | | | 20.65 | 240, 262, 270, 330 | NA | NA | 649.29 |

| **Trace at 450 nm, unsaponified extract (****Fig. 5B****):** | | | | | | | |
|---|---|---|---|---|---|---|---|
| *cis*-1-glycosyl-apo-8'-lycopene ester **(4)** | 0.71 | LY | 23.22 | 342, 426, 448, 478 | 53 | 57.1 | 401.3, n.d. |
| *cis*-1-glycosyl-apo-8'-lycopene ester **(5)** | 0.71 | LY | 24.12 | 342, 426, 448, 476 | 56.7 | 51 | 401.2, n.d. |
| 1-(6-C11)-glycosyl-apo-8'-lycopene **(6)** | 0.71 | LY | 24.75 | 344, 426, 450, 478 | 53 | 49.8 | 401.2, 749.2 |
| 1-(6-C11)-glycosyl-apo-8'-lycopene **(7)** | 0.71 | LY | 25.05 | 344, 424, 448, 476 | 58.3 | 48 | 401.2, 749.2 |
| 1-(6-C9)-glycosyl-apo-8'-lycopene **(8)** | 0.71 | LY | 25.5 | 344, 434, 448, 476 | 91.2 | 30 | 401.2, 721.1 |
| 1-(6-C9)-glycosyl-apo-8'-lycopene **(9)** | 0.71 | LY | 26.00 | 344, 426, 448, 476 | 90.1 | 17.3 | 401.2, 721,2 |
| 1-(6-C11)-glycosyl-apo-8'-lycopene **(10)** | 0.71 | LY | 27.09 | 344, 428, 452, 480 | 51.6 | 18.4 | 401.2, 581.1, 749.1 |
| 1-(6-C11)-glycosyl-apo-8'-lycopene **(11)** | 0.71 | LY | 27.55 | 424, 448, 476 | 71.8 | NA | 401.1, 581.1, 749.1 |
| Methyl 1-(6-C9)-glycosyl-apo-8'-lycopenoate **(12)** | 0.91 | O | 28.39 | 440, 466, 492 | 0.35 | NA | 445.1, 765.1 |
| Methyl 1-(6-C10)-glycosyl-apo-8'-lycopenoate **(13)** | 0.91 | O | 29.00 | 438, 466, 492 | <1 | NA | 445.1, 779.1 |

| | **TLC analysis** | | **HPLC** | **Spectral Properties** | | | |
|---|---|---|---|---|---|---|---|
| **Carotenoid** | **Rf** | **Colour** | **Rt** | **λmax (nm)** | **%III/II** | **% AB/AII** | **[M+H]+** |
| **Trace at 450 nm, unsaponified extract *(cont.)* (****Fig. 5B****):** | | | | | | | |
| Methyl 1-(6-C11)-glycosyl-apo-8'-lycopenoate **(14)** | 0.91 | O | 29.42 | 438, 466, 492 | <1 | NA | 445.1, 793.1 |
| Methyl 1-(6-C11)-glycosyl-apo-8'-lycopenoate **(15)** | 0.91 | O | 29.64 | 440, 466, 492 | 8 | NA | 445.1, 793.1 |
| 1-(6-C8)-glycosyl-apo-8'-lycopene **(16)** | 0.94 | Y | 30.02 | 430, 454, 484 | 68.4 | NA | 401.2, 707.2 |
| 1-(6-C9)-glycosyl-apo-8'-lycopene **(17)** | 0.94 | Y | 30.65 | 430, 454, 484 | 39.4 | NA | 401.2, 721.2 |
| 1-(6-C10)-glycosyl-apo-8'-lycopene **(18)** | 0.94 | Y | 31.22 | 428, 454, 484 | 47.4 | NA | 401.2, 735.2 |
| 1-(6-C11)-glycosyl-apo-8'-lycopene **(19)** | 0.94 | Y | 31.84 | 430, 454, 484 | 48.3 | NA | 401.2, 749.2 |
| 1-(6-C12)-glycosyl-apo-8'-lycopene **(20)** | 0.94 | Y | 32.49 | 430, 454, 484 | 59 | NA | 401.2, 763.2 |
| 1-(6-C13)-glycosyl-apo-8*-lycopene **(21)** | 0.94 | Y | 32.91 | 430, 454, 484 | 64.3 | NA | 401.2, 777.2 |
| 1-(6-C13)-glycosyl-apo-8'-lycopene **(22)** | 0.94 | Y | 33.27 | 430, 454, 484 | 66 | NA | 401.2, 777.2 |
| 1-(6-C14)-glycosyl-apo-8'-lycopene **(23)** | 0.94 | Y | 34.05 | 430, 454, 484 | 64.7 | NA | 401.2, 791.2 |
| 1-(6-C15)-glycosyl-apo-8'-lycopene **(24)** | 0.94 | Y | 35.03 | 430, 454, 484 | 66 | NA | 401.2, 805.2 |

**Table 2: Chromatographic and spectral properties of carotenoids detected in saponified extract of HU36 analysed by TLC, HPLC-PDA and LC/MS. Y: yellow (glycosyl-apolycopene); LY: light yellow; OR: orange carotenoid (methyl glycosyl-apolycopenoate), NA: not applicable; Nd: not detected.**

| | **TLC analysis** | | **HPLC** | **Spectral Properties** | | | |
|---|---|---|---|---|---|---|---|
| **Carotenoid** | **Rf** | **Colour** | **Rt** | **λmax (nm)** | **%III/I** | **% AB/AII** | **[M+H]+** |
| **Trace at 450 nm, saponified extract (****Fig. 5C****):** | | | | | | | |
| a*cis*-1-glycosyl-apo-8'-lycopene **(25)** | 0.21 | Light Yellow | 16.57 | 342, 424, 446, 474 | 29.8 | 47.8 | n.d. |
| *cis*-methyl 1-glycosyl-apo-8'-lycopenoate **(26)** | 0.21 | Light Orange | 19.3 | 358, 460, 488 | NA | 38.7 | n.d. |
| *cis*-1-glycosyl-apo-8'-lycepene **(27)** | 0.21 | Light Yellow | 20.2 | 344, 422, 446, 476 | 62.5 | 49 | 581.12 |
| *cis*-glycosyl-apo-8'-lycopene **(28)** | 0.21 | Light Yellow | 21.02 | 342, 428, 448, 476 | 55.3 | 28.3 | n.d. |
| *cis*-methyl 1-glycosyl-apo-8'-lycopenoate **(29)** | 0.34 | Orange | 24.10 | 356, 436, 464, 492 | <1 | 14.6 | 445.17, 624.90 |
| Methyl 1-glycosyl-apo-8'-lycopenoate **(30)** | 0.34 | Orange | 24.53 | 440, 466, 492 | 6.1 | NA | 625.06, 463.08, 445.09 |
| *cis*1-glycosyl-apo-8'-lycopene **(31)** | 0.38 | Yellow | 25.87 | 342, 426, 450, 478 | 66.3 | 11.2 | 581.02 |
| 1-glycosyl-apo-8'-lycopene **(32)** | 0.38 | Yellow | 27.08 | 428, 454, 484 | 64.2 | NA | 581,05, 419,06, 401.1 |

Comparison with the chromatographic and spectral properties of the diapocarotenoids found in *S. aureus* showed that the Bacillus carotenoids were not diaponeurosporene derivatives, but similarity to the *P. maritimus* methyl glucosyl-3,4-dehydro-apo-8'-lycopenonate was feasible.

Collectively the data suggests that the *Bacillus* carotenoids are apolycopene derivatives. Increased polarity after saponification treatment implies the presence of ester, not ether linked fatty acids presumably on glycosyl moieties. Furthermore, the ability of the C467 hydrolysis product to reside in the acid fraction demonstrates that it is carrying an additional carboxylic acid group which is absent in C455.

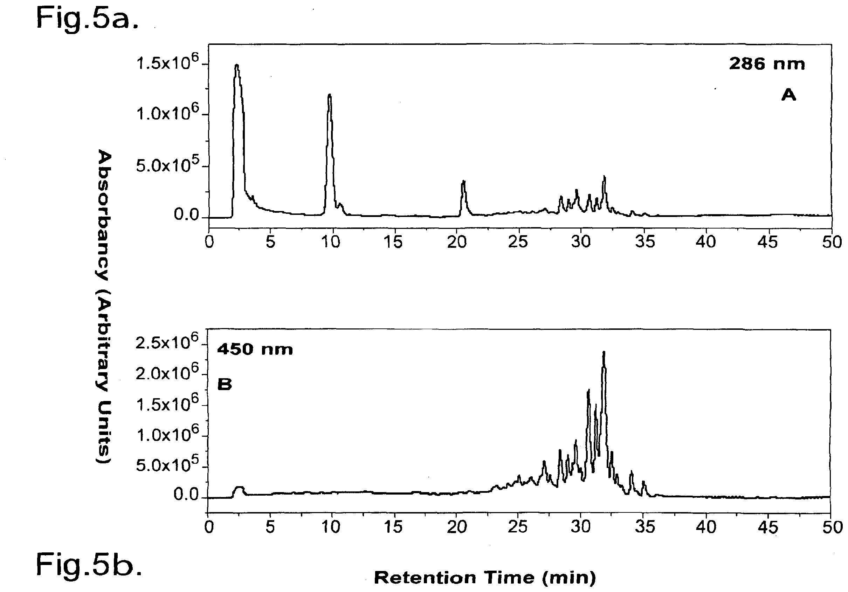

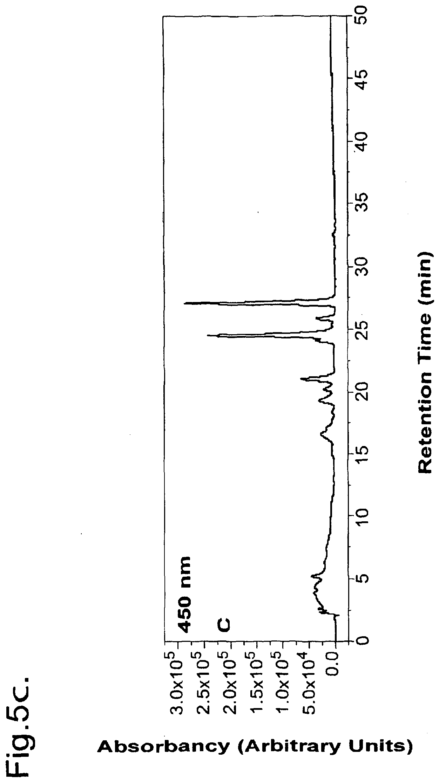

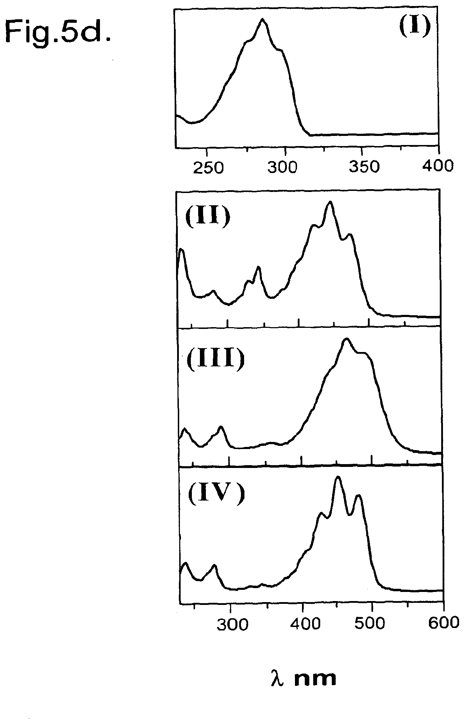

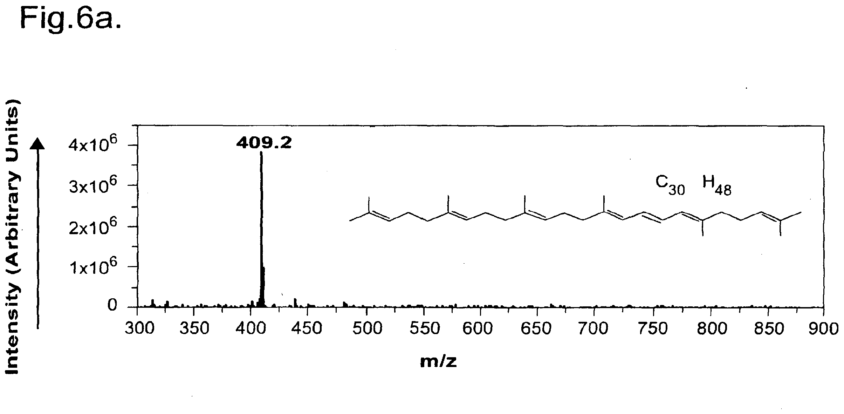

Following TLC, HPLC-PDA/MS analysis was performed. Figure 5 details the chromatographic profiles obtained with non-saponified extracts at 286 nm (A), 450 nm (B) and saponified extracts at 450 nm (C). Tables 1 and 2 document the chromatographic components, their UV/Vis and mass spectral properties. In non-saponified extracts a complex chromatogram was obtained. At 286 nm the predominant peak (1) at 9.8 min, had an absorption spectrum typical of phytoene (Figure 5 D-1), and adjacent was an apparent geometric isomer (peak 2). These compounds did not co-chromatograph with authentic C₄₀ phytoene but did possess identical properties to apophytoene and its isomers isolated from *Planococcus* and *Heliobacteria.* The identification of apophytoene in the bacterial extracts was confirmed by the mass spectrum of peaks 1 and 2 at 409.2 m/z (Fig. 6A), corresponding to a [M+H]⁺ for the parent ion of C₃₀ H₄₀. Besides apophytoene (1) and (2), Menaquinone-7 (3) was identified ([M+H]⁺ = 649.2).

The chromatographic profile at 450 nm of the the non-saponified methanolic extracts was complex with over 20 components (Fig.5B). These peaks could be categorised into several groups with identical UV/Vis spectra. Group II contained peaks 4 to 11, eluting between 23 to 27.5 min. These chromatographic components all had a visible spectral maximum of 452 nm (Fig. 5D-II), with persistence at 426 and 478 nm and pronounced *cis* peaks at 342 nm. The intensity of the *cis* peak was indicated by the A_{B}/A_{II} ratio provided in Table 1. It appears that higher the ratio and thus greater degree of *cis* geometric isomerisation the earlier is the retention time or more polar is the component. A mass of 401.2 [M+H]⁺ corresponding to an apolycopene skeleton was found in all these chromatographic components included in group II. Higher parent masses were also observed e.g. 749.2 [M+H]⁺ or 721.2 [M+H]⁺, but the presence of a hexose sugar (e.g. Δm/z of 162) and fatty acids (e.g. Δm/z of 186 for undecanoic acid) was revealed upon in source fragmentation of the higher mass parent ions. The fatty acids moieties found ranged from nonanoic, decanoic and undecanoic acids (Table 1). Thus the components of group II were designated as esterified glycosides of apolycopene existing in the *cis* geometric configuration predominantly.

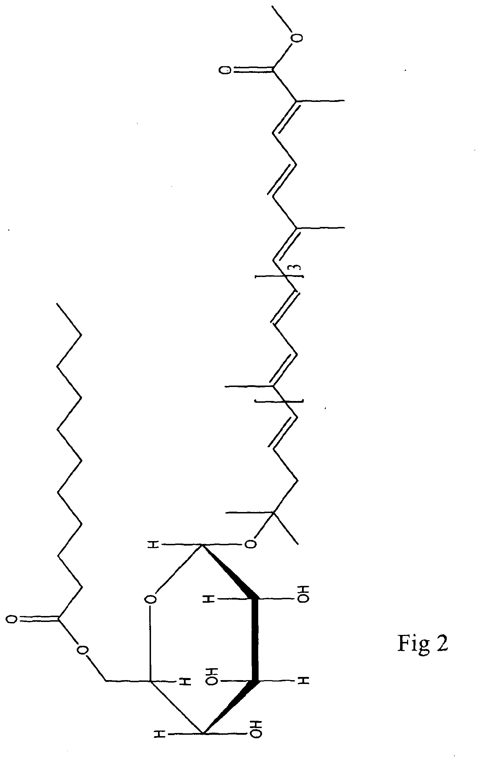

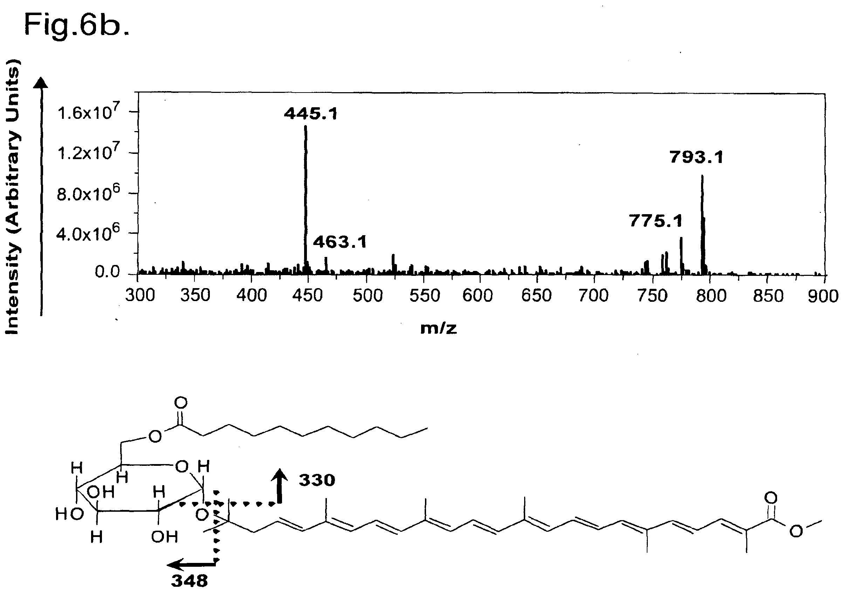

Chromatographic peaks 12 to 15, eluting 28 to 29.5 min represent group III. These chromatographic components all had UV/Vis maxima of 466 nm, persistence was reduced, with shoulders present at 438 and 492 nm. The mass of the parent carotenoid found in these peaks was determined to be 445.1 [M+H]⁺ instead of 401, indicating the presence of a methylated carboxylic acid. The occurrence of the acid group or more precisely the keto group explains the reduced persistence in the spectra and increased X maxima. The higher parent masses of these peaks could be accounted for by the presence of a hexose sugar and fatty acid derivatives, as differences in masses of 162 (hexose moiety) and 158-186 (C_{9:0}-C_{11:0} esters) were detected upon fragmentation (table 1). For example peak 15 was designated as methyl 1-(6-C_{11:0})-glycosyl-3,4-dehydroapo-8'-lycopenoate (Figure 2B), derived from a parent mass of 793.1 [M+H]⁺, carrying an hexose sugar (Δ*m*/*z* 162) esterified to undecanoic acid (Δ*m*/*z* 186) and attached to 445.1 [M+H⁺] dehydro-apo-8'-lycopenoate. Alternatively, this parent apocarotenoid can be obtained from 793.1 [M+H]⁺ through the loss of a hydroxyl group as water to 775.1, followed by the removal of hexose sugar {Δ*m*/*z* 144 = hexose-18) and fatty acids (Δ*m*/*z* 186 (C_{11:0})) yielding 463.1 from which the loss of another hydroxyl group as water would result in 445.7 [M+H]⁺ (Figure 6 B).

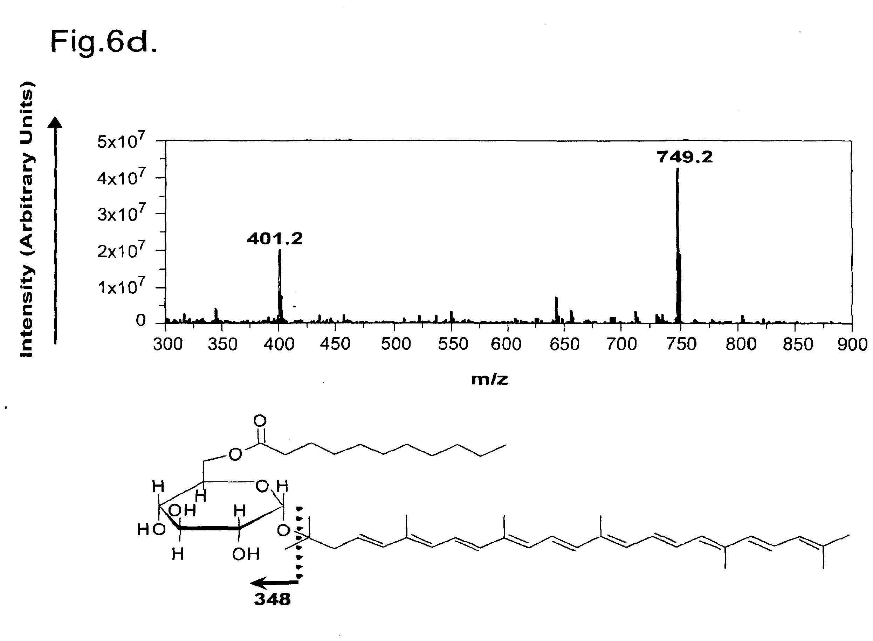

The peaks (17 to 24) in the chromatogram (Figure 5 B) are designated group IV where no methylated carboxylic acid forms of apolycopene occurred, instead the carotenoid skeleton apolycopene with a mass of 401.2 [M+H⁺] was found. The UV/Vis spectra were also more persistent and the maximum reduced to 454 nm, which was indicative of no keto group present. Using peak 19 as an example the parent molecule possessed a m/z of 749.2 [M+H]⁺, which yielded the apocarotenoid skeleton of 401.2 m/z through the excision of an esterfied-glycosyl component. This mass difference of 348 m/z is equivalent to the sum of a sugar (162 m/z) and fatty acid in this case saturated undecanoic acid (186 m/z), (Figure 6D). Similar analysis was performed on the rest of the yellow chromatographic components detected in group IV, permitting the identification of eight different esterified saturated fatty acids ranging from octanoic (C_{8:0}) to pentadecanoic (C_{15:0}) acid (Table 1). Several components (e.g. 21 and 22) showed identical spectral properties but different retention times. This was probably due to the presence of geometric isomers or the fatty acid present being branched (iso- or *anteiso*-) as this type of fatty acids are typical of those found in bacteria (24).

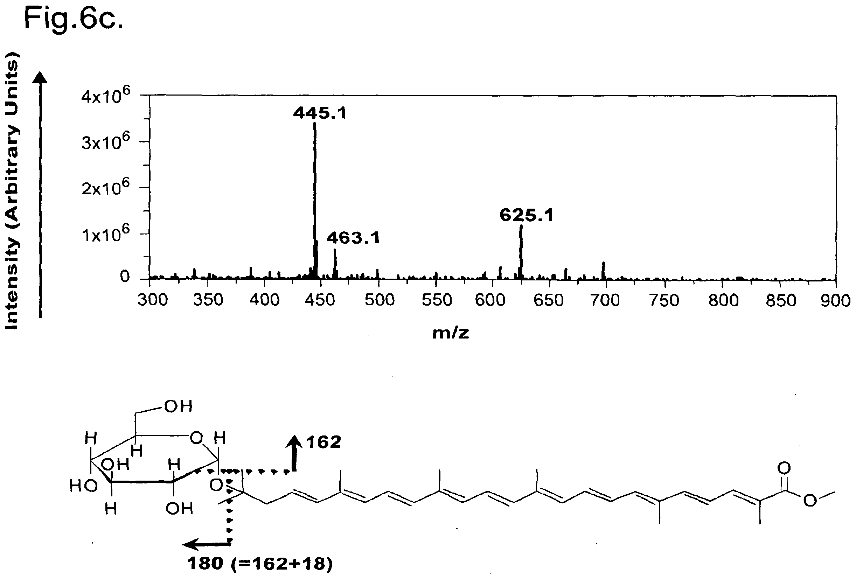

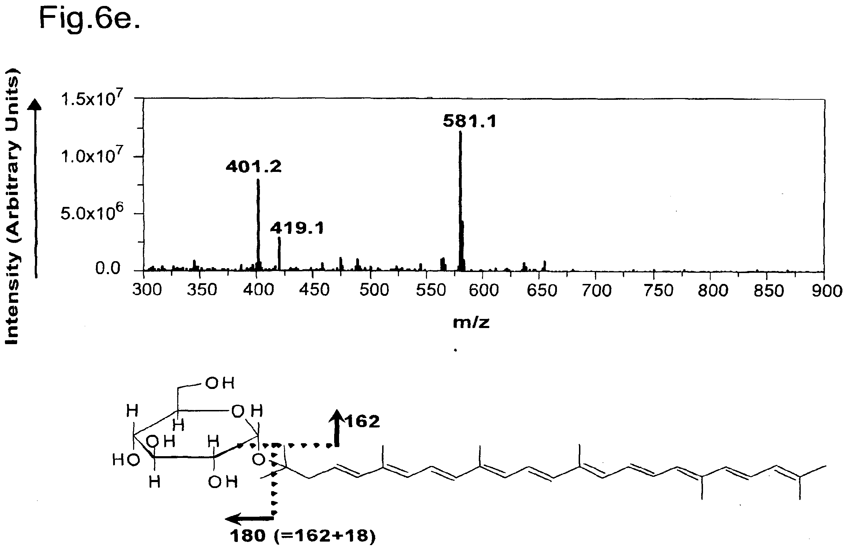

Saponification reduced the complexity of the chromatogram (Figure 5C). The chromatographic grouping of II, III, and IV were effectively identical as Figure 5B but without the presence of fatty acid moieties on the glycosylated apolycopene and apolycopenoate. These were clear from the mass spectra (Figure 6C and E). For example peak 30 and 32 possessed [M+H]⁺ of 625.1 (Figure 6C) and [M+H]⁺ 581.1 (Figure 6E) respectively, loss of hexose sugar (Δm/z of 162) (yielding 445.1 [M+H]⁺ for methyl-dehydro-apo-lycopenoate and 401.2 [M+H]⁺ for dehydro-apo-lycopene. Thus it is clear that the fatty acids are connected to the sugar residue by ester not ether bonds.

### 2.3. Identification of pathway intermediates using diphenylamine (DPA) treatment.

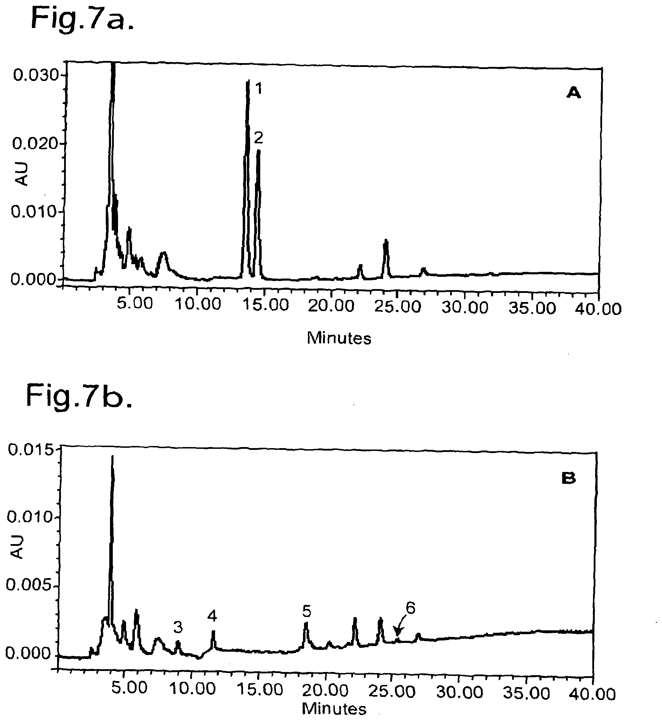

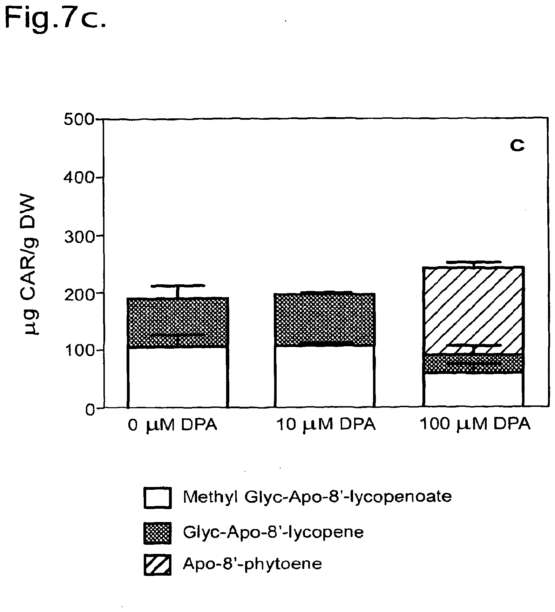

In order to identify intermediates in the *Bacillus* apocarotenoid biosynthetic pathway the known inhibitor of carotenoid desaturation, diphenylamine (DPA), was used. Treatments were carried out by seeding agar plates with DPA over a 0 to 100 µM range. The plates were inoculated with an aliquot (0.1 ml) of *Bacillus* culture broth taken at the mid-exponential growth phase. After 24 hr cultivation the biomass was harvested from the plates and carotenoids present in the material analysed. The most dramatic feature of the HPLC profiles (Figure 7A) was the increase in apophytoene (peak 1) and its geometric isomer (peak 2). In addition, the chromatogram recorded at 350 nm (Figure 7B) indicated the presence of chromatographic peaks unique to DPA treatment. These peaks were identified from their characteristic UV/Vis and Mass spectra as apophytofluene (peaks 3-5) and apo-ζ-carotene (peak 6). The C₃₀ apophytofluene had a visible spectrum with two persistent peaks at 350 and 365 nm, and a [M+H]⁺ of 403 m/z , whereas apo-ζ-carotene with two extra conjugated double bonds had a [M+H]⁺ of 405 m/z and a UV/Vis spectrum with clear persistence giving rise to two peaks at 400 and 425 nm. Interestingly, the amount of total carotenoid increased with the highest DPA concentration (Figure 7C) although it was not statistically significant. This was mainly due to the high concentration of apophytoene accumulating to 60% of the total carotenoid, compared to 1.2% in the wild type without DPA treatment (p<0.001) (Table 3).

**Table 3: Quantification by HPLC-PDA of coloured pigments glycosyl-apolycopene (yellow) and methyl glycosyl-apolucopenoate (orange) and the colourless precursor a pophyloene upon diphenylamine (DPA) treatment. DW: dry weight; Y-O: yellow/orange; W:while-creamy.**

| | | | **Carotenoid Content (µg/g DW)** | | | |
|---|---|---|---|---|---|---|
| | **Biomass (mg DW)** | **Colour** | **Total** | **Yellow** | **Orange** | **Apo-8'-phytoene** |
| **0 µM DPA** | 48.9±6.5 | Y-O | 186.8±46.4 | 78.2±27.8 | 106.3±19.5 | 2.3±0.9 |

| | **Biomass(mg DW)** | **Colour** | **Total** | **Yellow** | **Orange** | **Apo-8'-phytoene** |
|---|---|---|---|---|---|---|
| **10 µM DPA** | 40.8±3.8 | Y-O | 195.6±2.9 | 83.8±1.4 | 107.1±5.2 | 4.6±1.8 |
| **100 µM DPA** | 43.7±5.5 | W | 241.7±24.1 | 20.7±16.3 | 58.8±16.3 | 152.1±10.7 |

The amounts of the yellow (1-glycosyl-3,4-dehydro-apo-8'-lycopene) and orange (methyl 1-glycosyl-3,4 dehydro-apo-8' lycopenoate) pigments were reduced to a level about half that found in the untreated e.g. about 24% of the orange pigment accumulated at 100 µm DPA compared to 57% in the untreated (p<0.05). The increase in the occurrence of total carotenoid in the presence of DPA has been reported in other organisms, where it has been attributed to the elimination of regulation by end-product feedback inhibition or alternatively it is feasible that the pigments undergo further catabolism in these *Bacillus* species.

In some gram +ve bacteria squalene formed via the condensation of two farnesyl diphosphate (FPP) molecules and is the precursor for 4,4'-diapophytoene **(10).** Squalene was not detectable in the yellow/orange *Bacillus* species tested, using either LC-MS or GC-MS. Following DPA treatment the build up of precursors indicated no detectable squalene. The greatest similarity between the *Bacillus indicus* HU36 apocarotenoids and other bacteria is clearly *Planococcus*, which through NMR characterisation has been shown to form 8'-apocarotenoids. Therefore, it would appear that the *Bacillus* apocarotenoids in these yellow/orange strains are similar to those determined in Planococcus and 8'-apo in nature. To form 8'-apocarotenoids, geranylgeranyl diphosphate (C₂₀) and geranyl diphosphate (C₁₀) must be used as precursors instead of FPP.

### 2.3. Diapocarotenoid formation during the cellular development of Bacillus spp.

The ability of *Bacillus indicus* HU36 *indicus* and other orange/yellow species to form spores during development has been reported previously [25] along with a concurrent color change from yellow to orange. A detailed developmental staging was thus carried out to define pigment formation during sporulation. Cultivation in DSM media revealed the presence of vegetative cells at day 1 exclusively, by day 2 55% sporulation had occurred, plateauing until day 3 (Table 4).

**Table 4: Quantification by HPLC-PDA of glycosyl-apolycopene (yellow), methyl glycosyl-apolycopenoate (orange) 8'-apophytoene and the non-identified carotenoid eluting at 8 min (UNK-8 min) in HU36 cells grown either on DSM or TY media. Sporulation efficiency (% Sporulation) equals to (c.f.u of HRS/c.f.u. of Veg. Cells)x100. DW: dry weight; c.f.u: colony forming unit; HRS: heat resistant spores; n.d: not detected.**

| | | | | **Carotenoid (µg/g DW)** | | | | |
|---|---|---|---|---|---|---|---|---|
| **Media** | **Time (day)** | **% Sporulation** | **Biomass (mg DW)** | **Total** | **UNK-8 min** | **Glyc-Apo-8'-lycopene** | **Me Glyc-Apo-8'-lycopenoate** | **Apo-8'-phytoene** |
| **DSM** | 1 | 0 | 20.5 | 111.03±13.8 | 1.23±0.06 | 43.6±13.7 | 66.23±0.06 | n.d |
| | 2 | 50.7±8.97 | 19.2 | 175.5±41.5 | 1.96±0.42 | 39.6±1.8 | 133.8±39.3 | n.d |
| | 3 | 60.2±10.5 | 17.5 | 88.8±2.01 | 1.58±0.43 | 10.7±0.4 | 76.5±2.04 | n.d |
| **TY** | 1 | 0 | 37.3 | 123.1±34.1 | 3.9±0.9 | 110.03±45.9 | nd | 18.1±0.00 |
| | 2 | 0.03±0.01 | 120 | 130.2±3.3 | 15.9±6.3 | 86.5±2.5 | 14.1±0.4 | 13.71±0.04 |
| | 3 | 0.03±0.005 | 69 | 34.1±7.8 | 24.3±0.3 | 34.1±7.8 | 11.5±2.8 | 12.2±3.3 |

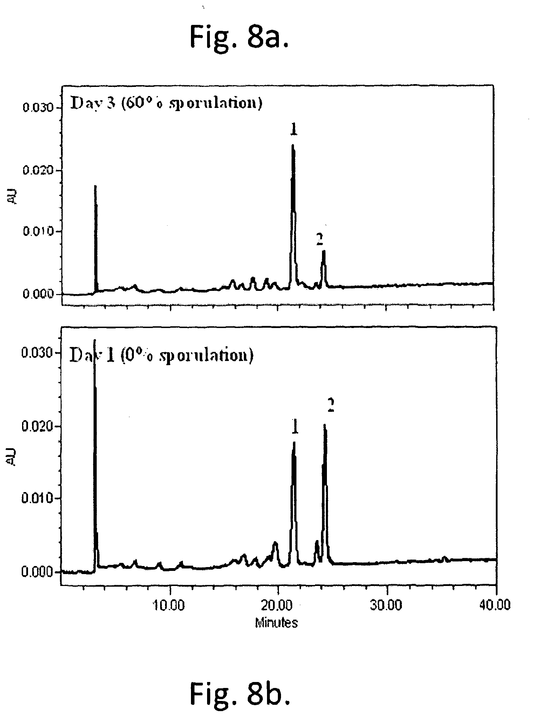

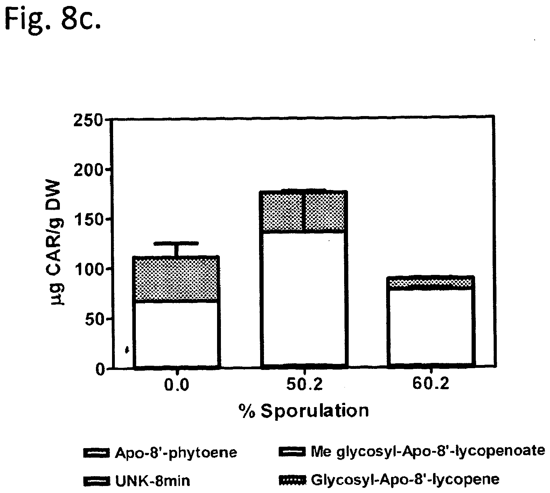

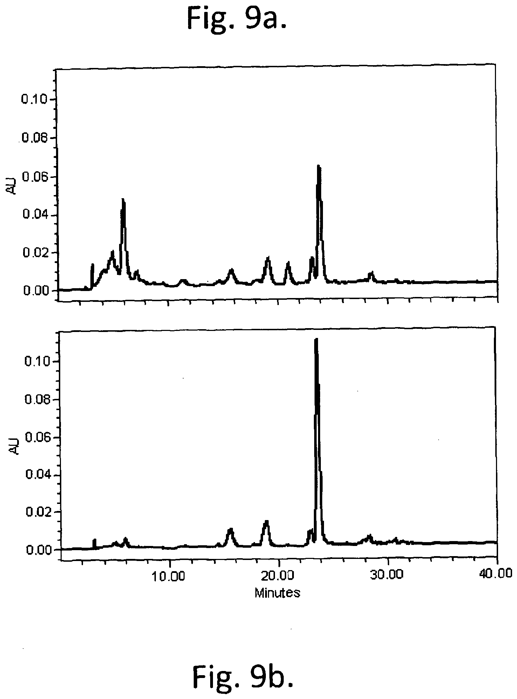

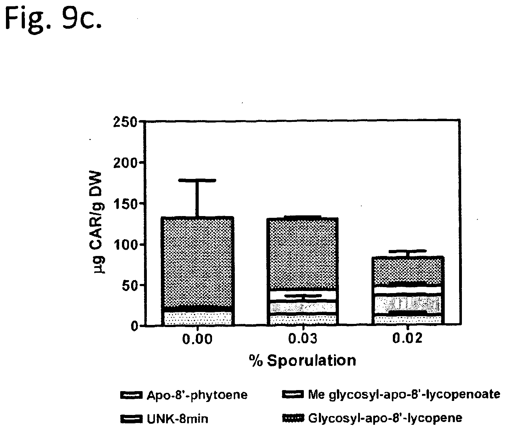

The content of the yellow pigment 1-glycosyl-3,4-dehydro-apo-8'-lycopene (Compound 19) was comparatively constant over development. However, the orange pigment methyl-1-glycosyl-3-4, dehydro-apo 8'-lycopenoate (Compound 15) significantly increased (p<0.05) about 43% with the onset of sporulation (Figure 8; Table 4). In TY media sporulation did not occur and interestingly the orange pigment methyl 1-glycosyl-3, 4-dehydro-apo-8' lycopenoate (Compound 15) did not accumulate (Figure 9; Table 4). These data suggest that apocarotenoid formation in these pigmented *Bacilli* is in part under developmental regulation with the formation of diapocarotenoid methyl 1-glycosyl-3, 4 dehydroapo-8'-lycopenoate strictly related by this developmental event.

### 3. Conclusions

A combination of biochemical techniques has been used in the present study to demonstrate the presence of C₃₀ apocarotenoid derivatives in certain pigmented *Bacillus spp.* The yellow pigment 1-glycosyl-3-4-dehydro-apolycopene ester predominates in vegetative cells, while the formation of the orange pigment methyl 1-glycosyl-3,4-dehydro-8'-apolycopenate ester is enhanced during sporulation. Thus developmental regulation of the biosynthetic pathway appears to occur in these *Bacillus spp.* Apophytoene was the first genuine C₃₀ carotenoid detected in these *Bacillus spp.*

In summary, useful carotenoids present in the yellow /orange pigmented *Bacillus spp* such as *Bacillus indicus* HU36 (NCIMB 41361) have been characterised.

### 5. References

[1] G. Britton, S. Liaaen-Jensen and H. Pfander. in Carotenoids, Birkhauser Verlag, Basel 2004.
[2] N. Barber, BJU Int 91 (2003) 307-9.
[3] E. Giovannucci, J Natl Cancer Inst 91 (1999) 317-31.
[4] E. Giovannucci, A. Ascherio, E.B. Rimm, M.J. Stampfer, G.A. Colditz and W.C. Willett, J Natl Cancer Inst 87 (1995) 1767-76.
[5] J.A. Olson and O. Hayaishi, Proc Natl Acad Sci U S A 54 (1965) 1364-70.
[6] D.M. Snodderly, Am J Clin Nutr 62 (1995) 1448S-1461S.
[7] P.M. Bramley. in (Johnson, I. and Williamson, G., eds.) Phytochemical functional foods, CRC Press, Boca Raton 2003, pp. 253-279.
[8] H. Kleinig and R. Schmitt, Zeitschrift Fur Naturforschung C-a Journal of Biosciences 37 (1982) 758-760.
[9] H. Kleinig, R. Schmitt, W. Meister, G. Englert and H. Thommen, Zeitschrift Fur Naturforschung C-a Journal of Biosciences 34 (1979) 181-185.
[10] L. Tao, A. Schenzle, J.M. Odom and Q. Cheng, Appl Environ Microbiol 71 (2005) 3294-301.
[11] B.H. Davies and R.F. Taylor, Canadian Journal of Biochemistry 60 (1982) 684-692.
[12] S. Takaichi, K. Inoue, M. Akaike, M. Kobayashi, H. Oh-oka and M.T. Madigan, Arch Microbiol 168 (1997) 277-81.
[13] S. Takaichi, H. Oh-Oka, T. Maoka, D.O. Jung and M.T. Madigan, Arch Microbiol 179 (2003) 95-100.
[14] J.H. Marshall and G.J. Wilmoth, Journal of Bacteriology 147 (1981) 914-919.
[15] J.H. Marshall and G.J. Wilmoth, Journal of Bacteriology 147 (1981) 900-913.
[16] A. Pelz, K.P. Wieland, K. Putzbach, P. Hentschel, K. Albert and F. Gotz, Journal of Biological Chemistry 280 (2005) 32493-32498.
[17] H.A. Hong, J.M. Huang, R. Khaneja, L.V. Hiep, M.C. Urdaci and S.M. Cutting, J Appl Microbiol 105 (2008) 510-20.
[18] R. Khaneja, L. Perez-Fons, S. Fakhry, L. Baccigalupi, S. Steiger, E. To, G. Sandmann, T.C. Dong, E. Ricca, P.D. Fraser and S.M. Cutting, J Appl Microbiol (2009).
[19] W.L. Nicholson and P. Setlow, SPORULATION GERMINATION AND OUTGROWTH 1990.
[20] R.F. Taylor and B.H. Davies, Canadian Journal of Biochemistry and Cell Biology 61 (1983) 892-905.
[21] P.D. Fraser, M.E. Pinto, D.E. Holloway and P.M. Bramley, Plant Journal 24 (2000) 551-8.
[22] A.J. Aasen, G.W. Francis and S. Liaaen-Jensen, Acta Chem Scand 23 (1969) 2605-2615.
[23] G. Britton, S. LiaaenJensen and H. Pfander, CAROTENOIDS HANDBOOK, Birkhauser Verlag Ag 2004.
[24] T. Kaneda, J Bacteriol 93 (1967) 894-903.
[25] L.H. Duc, P.D. Fraser, N.K.M. Tam and S.M. Cutting, Fems Microbiology Letters 255 (2006) 215-224.

## Claims

1. A carotenoid compound of Formula (2): 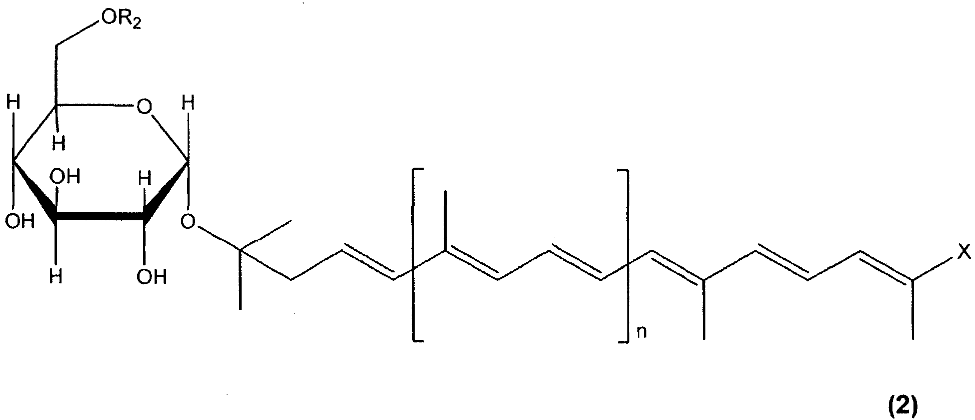 wherein X is methyl or COOR wherein R is independently selected from methyl, ethyl, methylethyl, propyl, isopropyl, butyl, isobutyl, sec-butyl, and tert-butyl; and n is selected from zero, 1, 2, and 3; and R₂ is independently selected from octadecanoic acid, nonadecanoic acid, decanoic acid, undecanoic acid, dodecanoic acid, tridecanoic acid, tetradecanoic acid and pentadecanoic acid.

2. A compound according to claim 1 of Formula (1): 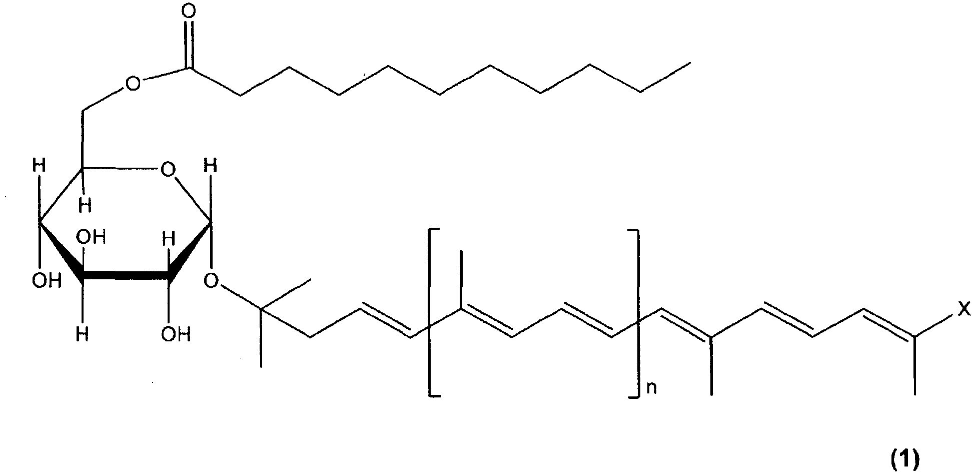 wherein X is independently selected from methyl and COOR wherein R is independently selected from methyl, ethyl, methylethyl, prop-1-yl, butyl, isobutyl, *sec*-butyl, and *tert*-butyl; and n is selected from zero 1, 2, and 3.

3. A compound according to claim 2 wherein X is methyl and n is 3.

4. A compound according to claim 2 wherein X is COOR and R is independently selected from methyl, ethyl, methylethyl, propyl, isopropyl, butyl, isobutyl, *sec*-butyl, and *tert*-butyl; and n is 3.

5. A compound according to claim 4 wherein R is methyl.

6. Use of at least one isolated compound according to any one of claims 1 to 5 as a colouring agent for a foodstuff.

7. Use according to claim 6 wherein the foodstuff is a pro-biotic beverage, or a non-liquid foodstuff.

8. Use according to claim 6 wherein the foodstuff is selected from snack bars, cereals, buns, muffins, biscuits, cakes, pastries, processed vegetables, sweets (candies), processed sauces, dips and spreads, confectionery, probiotic formulations including yoghurts, beverages, plant oil-based liquids, animal fat-based liquids, frozen confections, and cheeses.

9. Use according to claim 6 wherein the foodstuff is selected from yoghurts, cheeses, soft beverages such as squashes (e.g. orange and lemon), nutritional beverages and teas.

10. Use of at least one isolated compound according to any one of claims 1 to 5 in a foodstuff as an antioxidant.

11. Use of a compound according to Formula (2): 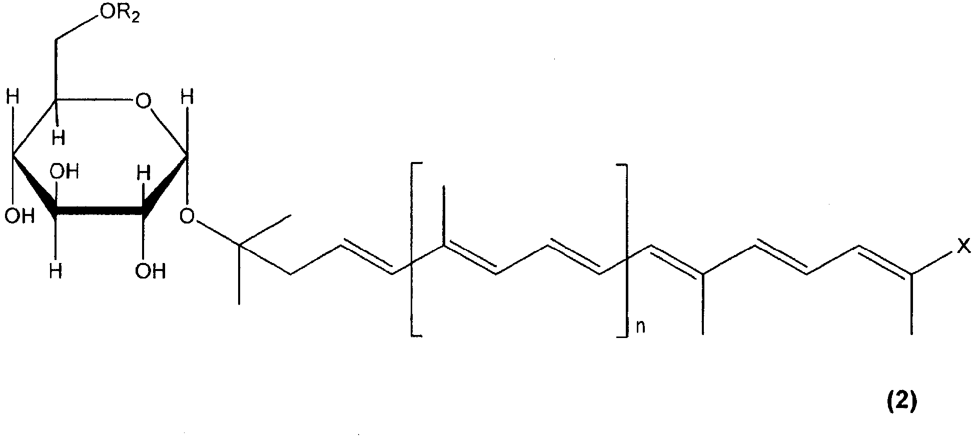 as a colouring agent for a foodstuff wherein R₂ is H; and X, COOR and n are all as defined according to claim 1.

12. Use of a compound according to any one of claims 1 to 5 in a composition for use on human skin as a cosmetic or for use in a foodstuff as an antioxidant.

## Patentansprüche

1. Carotinoidverbindung der Formel (2): 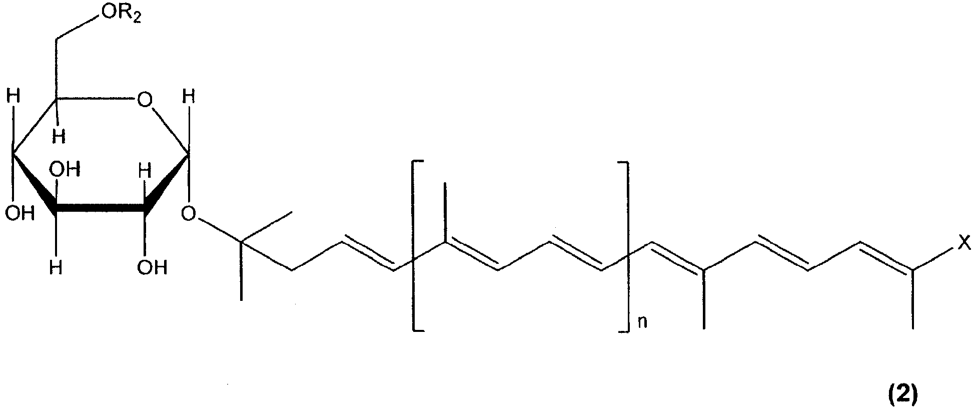 wobei X Methyl oder COOR ist, wobei R unabhängig aus Methyl, Ethyl, Methylethyl, Propyl, Isopropyl, Butyl, Isobutyl, sek.-Butyl und tert.-Butyl ausgewählt ist; und n aus null, 1, 2 und 3 ausgewählt ist und R₂ unabhängig aus Octadecansäure, Nonadecansäure, Decansäure, Undecansäure, Dodecansäure, Tridecansäure, Tetradecansäure und Pentadecansäure ausgewählt ist.

2. Verbindung nach Anspruch 1 der Formel (1): 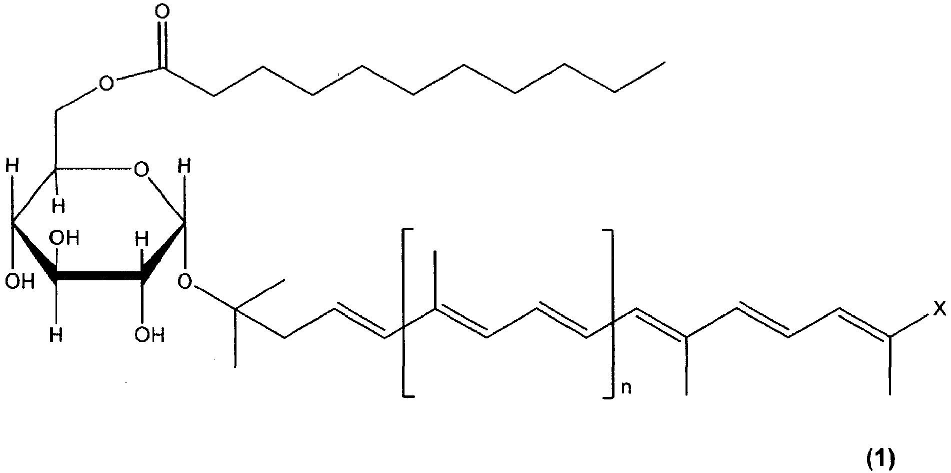 wobei X unabhängig aus Methyl und COOR ausgewählt ist, wobei R unabhängig aus Methyl, Ethyl, Methylethyl, Prop-1-yl, Butyl, Isobutyl, *sek*.-Butyl und *tert*.-Butyl ausgewählt ist; und n aus null, 1, 2 und 3 ausgewählt ist.

3. Verbindung nach Anspruch 2, wobei X Methyl ist und n 3 ist.

4. Verbindung nach Anspruch 2, wobei X COOR ist und R unabhängig aus Methyl, Ethyl, Methylethyl, Propyl, Isopropyl, Butyl, Isobutyl, *sek*.-Butyl und *tert*.-Butyl ausgewählt ist und n 3 ist.

5. Verbindung nach Anspruch 4, wobei R Methyl ist.

6. Verwendung von mindestens einer isolierten Verbindung nach einem der Ansprüche 1 bis 5 als ein Farbstoff für ein Nahrungsmittel.

7. Verwendung nach Anspruch 6, wobei das Nahrungsmittel ein probiotisches Getränk oder ein nichtflüssiges Nahrungsmittel ist.

8. Verwendung nach Anspruch 6, wobei das Nahrungsmittel aus Snack-Riegeln, Getreideflocken, Brötchen, Muffins, Keksen, Kuchen, Gebäck, Fertiggemüse, Süßigkeiten (Bonbons), Fertigsoßen, Dips und Aufstrichen, Süßwaren, probiotischen Rezepturen, einschließlich von Joghurts, Getränken, Flüssigkeiten auf Pflanzenölbasis, Flüssigkeiten auf Tierfettbasis, gefrorenem Konfekt und Käse ausgewählt ist.

9. Verwendung nach Anspruch 6, wobei das Nahrungsmittel aus Joghurts, Käse, Erfrischungsgetränken, wie Schorlen (z. B. Orange und Zitrone), nahrhaften Getränken und Tee ausgewählt ist.

10. Verwendung von mindestens einer isolierten Verbindung nach einem der Ansprüche 1 bis 5 in einem Nahrungsmittel als ein Antioxidans.

11. Verwendung einer Verbindung gemäß Formel (2): 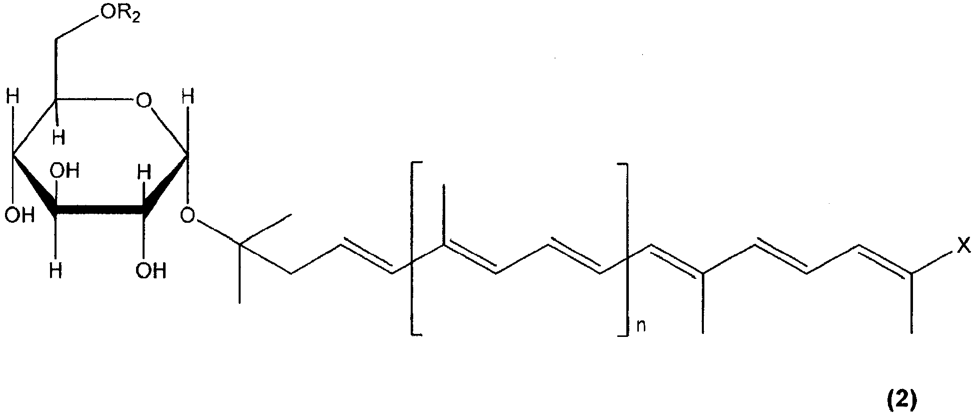 als ein Farbstoff für ein Nahrungsmittel, wobei R₂ H ist und X, COOR und n alle wie gemäß Anspruch 1 definiert sind.

12. Verwendung einer Verbindung nach einem der Ansprüche 1 bis 5 in einer Zusammensetzung zur Verwendung auf menschlicher Haut als ein Kosmetikum oder zur Verwendung in einem Nahrungsmittel als ein Antioxidans.

## Revendications

1. Composé caroténoïde de formule (2) : 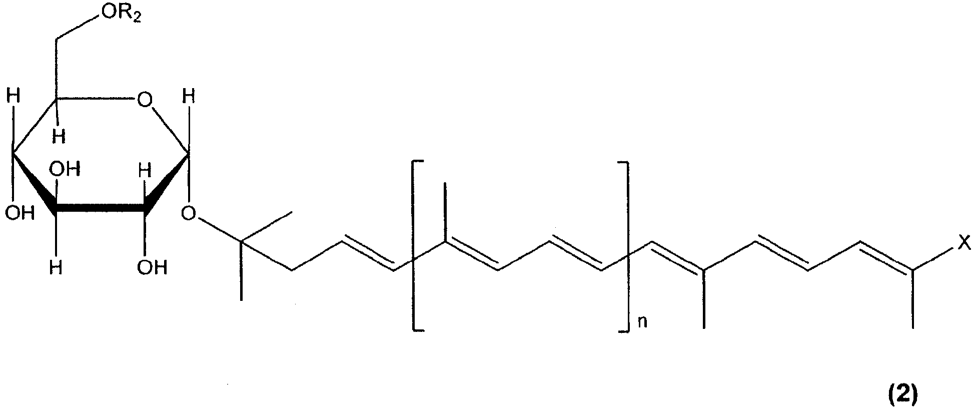 dans laquelle X représente un groupe méthyle ou COOR où R est indépendamment choisi parmi un groupe méthyle, éthyle, méthyléthyle, propyle, isopropyle, butyle, isobutyle, sec-butyle et tert-butyle ; et n est choisi parmi zéro, 1, 2 et 3 ; et R₂ est indépendamment choisi parmi un groupe acide octadécanoïque, acide nonadécanoïque, acide décanoïque, acide undécanoïque, acide dodécanoïque, acide tridécanoïque, acide tétradécanoïque et acide pentadécanoïque.

2. Composé selon la revendication 1 de formule (1) : 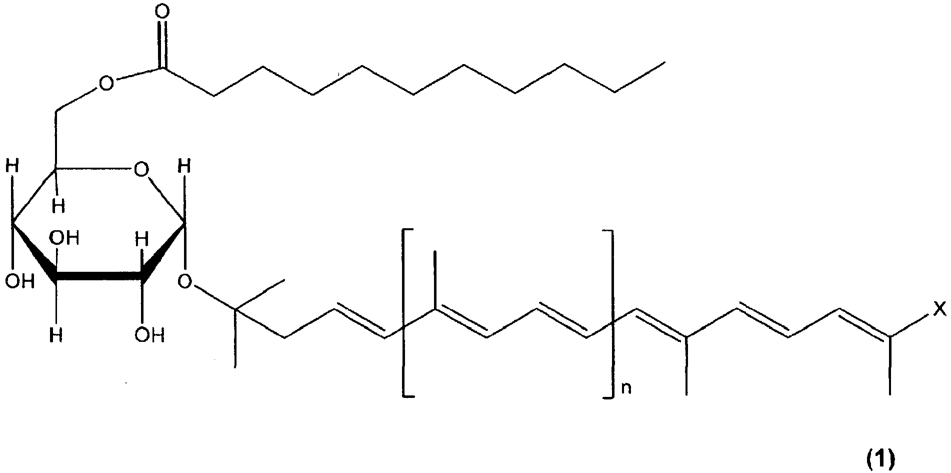 dans laquelle X est indépendamment choisi parmi un groupe méthyle et COOR où R est indépendamment choisi parmi un groupe méthyle, éthyle, méthyléthyle, prop-1-yle, butyle, isobutyle, *sec*-butyle et *tert*-butyle ; et n est choisi parmi zéro, 1, 2 et 3.

3. Composé selon la revendication 2, X représentant un groupe méthyle et n valant 3.

4. Composé selon la revendication 2, X représentant un groupe COOR et R étant indépendamment choisi parmi un groupe méthyle, éthyle, méthyléthyle, propyle, isopropyle, butyle, isobutyle, *sec*-butyle et *tert*-butyle ; et n vaut 3.

5. Composé selon la revendication 4, R représentant un groupe méthyle.

6. Utilisation d'un composé isolé selon l'une quelconque des revendications 1 à 5 comme agent colorant pour aliment.

7. Utilisation selon la revendication 6, ledit aliment étant une boisson probiotique ou un aliment non liquide.

8. Utilisation selon la revendication 6, ledit aliment étant choisi parmi les casse-croûtes, les céréales, les petits pains, les muffins, les biscuits, les gâteaux, les pâtisseries, les légumes transformés, les sucreries (bonbons), les sauces transformées, les sauces à tremper et les pâtes à tartiner, les confiseries, les formulations probiotiques dont les yaourts, les boissons, les liquides à base d'huile végétale, les liquides à base de graisse animale, les confiseries congelées et les fromages.

9. Utilisation selon la revendication 6, ledit aliment étant choisi parmi les yaourts, les fromages, les boissons sans alcool tels que les concentrés de jus de fruits (par ex. orange et citron), les boissons nutritives et les thés.

10. Utilisation d'au moins un composé isolé selon l'une quelconque des revendications 1 à 5 dans un aliment comme antioxydant.

11. Utilisation d'un composé selon la formule (2) : 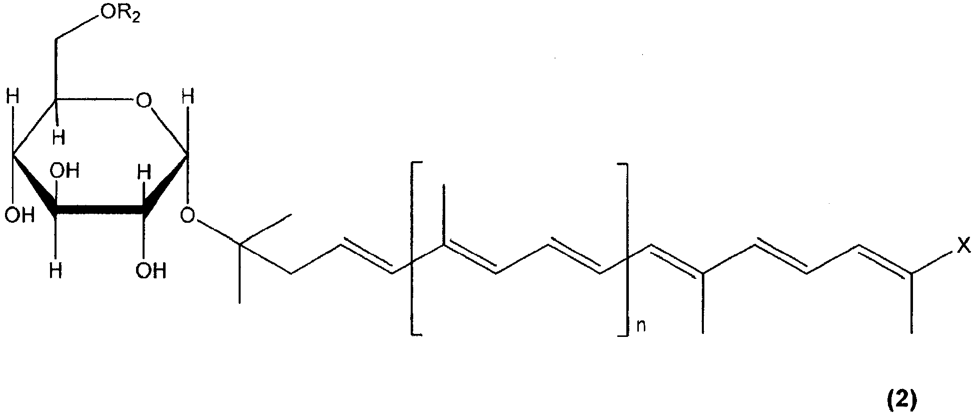 comme agent colorant pour aliment dans laquelle R₂ représente un atome H ; et X, COOR et n sont tous tels qu'ils sont définis selon la revendication 1.

12. Utilisation d'un composé selon l'une quelconque des revendications 1 à 5 dans une composition pour utilisation sur une peau humaine comme produit cosmétique ou pour utilisation dans un aliment comme antioxydant.
